# EUROPÄISCHE PATENTSCHRIFT

(11) **EP 0 294 626 B1**
(45) Veröffentlichungstag und Bekanntmachung des Hinweises auf die Patenterteilung: **15.12.1993**
(21) Anmeldenummer: 88108011.3
(22) Anmeldetag: 19.05.1988
(51) Int. Cl.: A61B 1/12

(54) **Endoskop**
Endoscope
Endoscope

(30) Priorität: 10.06.1987 DE 3719250
(43) Veröffentlichungstag der Anmeldung: 14.12.1988
(73) Patentinhaber: Kellner, Hans-Jörg, Dr.med., D-86356 Neusäss (DE); KOGLEK UND STARCK GMBH Ingenieurbüro für Medizintechnik, D-82291 Mammendorf (DE)
(72) Erfinder: Kellner, Hans-Jörg, Dr.med., D-8902 Neusäss (DE)
(74) Vertreter: Charrier, Rolf, Dipl.-Ing.

(56) Entgegenhaltungen:
- EP-A- 0 184 778
- WO-A-85/02101
- FR-A- 2 331 357
- GB-A- 2 167 668
- US-A- 4 445 892

## Beschreibung

Die Erfindung betrifft ein Endoskop nach dem Oberbegriff des Anspruchs 1.

Endoskope in der Medizin dienen in erster Linie der Diagnostik. Der mindestens eine Lichtleiter wird an eine Kaltlichtquelle angeschlossen und leitet deren Licht an die Spitze des Endoskopschlauches. Über den Spülkanal wird eine Spülflüssigkeit der Spitze zugeführt, die dort die Aufgabe hat, die Spitze von Körperflüssigkeit, wie z.B. Blut, freizuspülen. Das vom Bildleiter aufgenommene Bild wird einem Linsensystem am anderen Ende des Endoskopschlauches zugeführt. Es ist bekannt, zur Vornahme kleinerer Eingriffe am Endoskopkopf chirurgische Werkzeuge anzubringen, welche von außen bedienbar sind.

Die FR-A-2 331 357 beschreibt beispielsweise ein Endoskop zur Verwendung bei der Magenspülung mit einem Katheter, welches Sang- und Spülkanäle sowie Licht- und Bildleiter beinhaltet. Zur besseren Bedienbarkeit des Instruments zweigen die Kanäle sowie der Lichtleiter vor dem arztseitigen Ende des Instruments zur Seite ab. Damit wird ein von diesen Vorrichtungen ungestörter Einblick in das Okular ermöglicht. Die Vorrichtung kann nur als Ganzes sterilisiert werden, dabei ergeben sich wegen der langen und engen Kanäle große Schwierigkeiten sowohl bei der Sterilisation als auch bei der vorhergehenden Reinigung. Ein Wegwerfen der gesamten Vorrichtung nach einmaliger Benutzung ist wegen der teuren optischen Bauteile nicht möglich.

Ein weiterer Verwendungszweck von Endoskopen tritt bei chirurgischen Eingriffen, insbesondere bei Eingriffen an den unteren Extremitäten, auf. Hierbei ist die Beseitigung von Lungenembolien ein ernstes Problem. Ursprungsort der embolisierenden Thromben sind meist die Beinvenen. Diese Thromben werden über das Herz und die Pulmonalarterien in die Lungenstrombahnen gepreßt, welche hierdurch verstopfen. Bei leichten Lungenembolien wendet man eine Lysetherapie an, bei welcher die verstopfenden Thromben sich auflösen sollen. Bei schweren Lungenembolien ist eine Operation zur chirurgischen Beseitigung der Thromben erforderlich, bei welcher der Patient an eine Herz-Lungenmaschine angeschlossen werden muß. Insbesondere bei schweren Lungenembolien ist die Sterblichkeitsrate sehr hoch. Die chirurgische Beseitigung von Thromben kann nur in herzchirurgischen Zentren vorgenommen werden. Hierbei ist die erste Voraussetzung, daß der Patient einen Transport in ein solches Zentrum überlebt.

Neben dem hohen Sterberisiko ist beachtlich, daß beide Behandlungsverfahren relativ teuer sind. Eine Lysebehandlung, die üblicherweise über eine Woche hinweg durchgeführt wird, kostet etwa DM 15.000. Die Kosten einer Operation unter Verwendung einer Herz-Lungenmaschine betragen etwa DM 25.000 bis DM 30.000. Es wurden deshalb in der Vergangenheit verschiedene Endoskope zur Entfernung von Thromben aus Blutgefäßen entwickelt.

Die US-A-4,445,892 beschreibt ein Endoskop mit einem Lichtleiter, einem Bildleiter, einem Spülkanal und einem an eine Saugpumpe anschließbaren Saugkanal. Hierbei münden der Licht- und der Bildleiter sowie der Spül- und der Saugkanal an der Spitze des patientenseitigen Endteils des Endoskopschlauches. Dieses Endteil ist von einem delatierbarem Ballon umgeben. Weiterhin ist ein über die Spitze dieses Endteils überstehender Katheter vorgesehen, der an seinem Ende einen zweiten delatierbaren Ballon aufweist. Im Gebrauch wird der zu entfernende Thrombus zwischen diese beiden Ballons eingeschlossen und abgesaugt. Wegen der vielen langen und engen Rohrleitungen, welche zudem nach Gebrauch teilweise verstopft sein können, ist eine Sterilisation des hier beschriebenen Endoskops nur unter großen Schwierigkeiten möglich. Außerdem müssen vor der eigentlichen Sterilisation sämtliche Kanäle sorgfältig gereinigt werden, was mit den gleichen Schwierigkeiten verbunden ist.

Die WO-A-85/02101 beschreibt ein aus einem Handgriff und einem Katheter bestehendes Endoskop, wobei der Handgriff die Okularoptik enthält. Handgriff und Katheter sind voneinander lösbar. Der Katheter beinhaltet sämtliche Kanäle sowie die Licht- und Bildleiter sowie das am Ende des Bildleiters angebrachte Objektiv. Auf diese Weise kann zwar der Handgriff separat sterilisiert werden, jedoch treten bei der Sterilisation des Katheters die oben genannten Probleme auf. Der Katheter kann jedoch auch nicht nach jedem Gebrauch weggeworfen und durch einen neuen ersetzt werden, da er teure optische Elemente, nämlich Licht- und Bildleiter sowie das Objektiv, enthält.

Ausgehend von dem in der US-A-4,445,892 beschriebenen Stand der Technik stellt sich die Aufgabe, das Endoskop so auszubilden, daß seine Sterilität und Sterilisierbarkeit auf einfache und billige Weise gewährleistet ist.

Gelöst wird diese Aufgabe mit den kennzeichnenden Merkmalen des Anspruches 1. Vorteilhafte Ausgestaltungen sind den Unteransprüchen entnehmbar.

Die endoskopische Auffindung der Thromben und deren Ektomie durch das gleiche Gerät ist praktisch in jedem modern ausgestatteten Krankenhaus durchführbar. Schon bei den ersten Anzeichen einer Lungenembolie ist es möglich, Diagnose und Therapie sofort einzuleiten, wozu am Patienten lediglich ein geringer Eingriff an einer der Halsvenen erforderlich ist. Damit entfällt der meist lebensgefährliche Transport des Patienten zu einem herzchirurgischen Zentrum. Außerdem wird der Patient nicht mit einem großen chirurgischen Eingriff belastet, wie dies bislang bei Operationen unter Verwendung einer Herz-Lungenmaschine der Fall war. Da das Gerät mehrfach verwendbar ist, sind auch die Kosten zur Beseitigung einer Lungenembolie wesentlich geringer, auch im Vergleich zu einer Lysebehandlung.

Ein Ausführungsbeispiel wird nachfolgend anhand der Zeichnungen näher erläutert. Es zeigen:
- Fig. 1: Eine Seitenansicht des vorderen Bereichs des Endoskops;
- Fig. 2: einen Schnitt durch den Endoskopschlauch;
- Fig. 3: die schematische Darstellung eines Patienten zur Erläuterung des Vorgehens bei einer Lungenembolie und bei einer Beseitigung eines Thrombus in einer Beinvene und
- Fig. 4: die perspektivische Ansicht eines gemeinsamen Kupplungsstücks, an welchem die Kanäle außerhalb des Patienten münden.

Der Endoskopschlauch 1 weist in bekannter Weise zwei Lichtleiter 2, 3 auf, welche außerhalb des Patienten an eine Kaltlichtquelle 4 angeschlossen sind. Diese Lichtleiter 2, 3 münden an der verrundeten Spitze 5 des patientenseitigen Endteils 6 des Endoskopschlauches 1. Zwischen den beiden Lichtleitern 2, 3 verläuft der Bildleiter 8, der von der Spitze 5 zu einer Betrachtungsoptik 7 führt. In bekannter Weise weist der Endoskopschlauch 1 einen an der Spitze 5 mündenden Spülkanal 9 auf. Über zwei nicht dargestellte Bowdenzüge ist es möglich, das Endteil 6, das etwa 10 cm lang ist, um 120° bilateral zu schwenken.

Die vorbeschriebenen Merkmale des Endoskops und seines Schlauches sind bekannt. Die beiden Lichtleiter 2, 3 weisen einen Durchmesser von jeweils etwa 1,0 mm auf. Der Bildleiter 8 ist etwa 1,2 mm stark. Der Durchmesser des Spülkanals 9 beträgt etwa 1,0 mm.

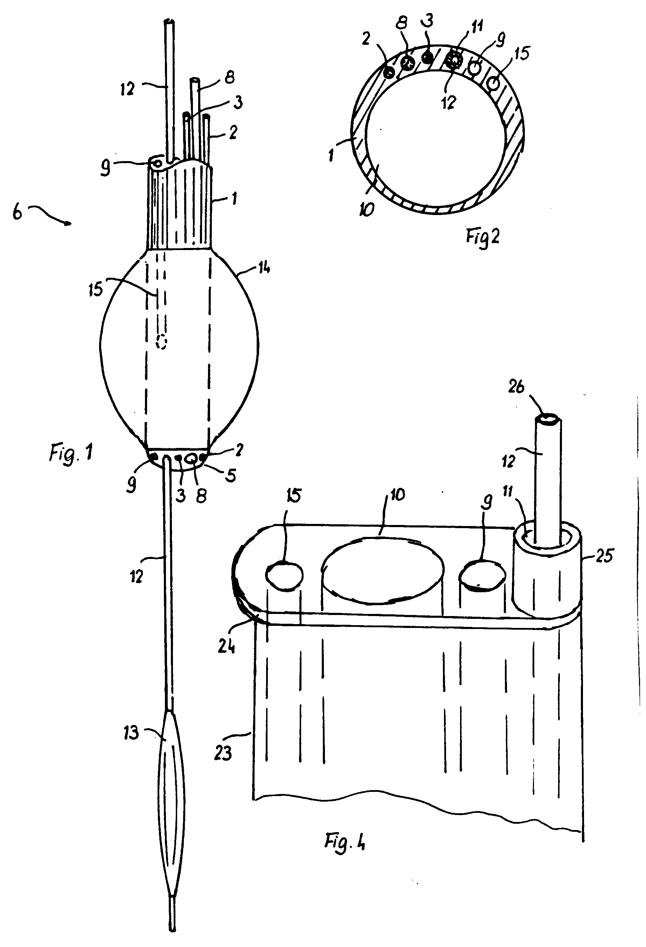

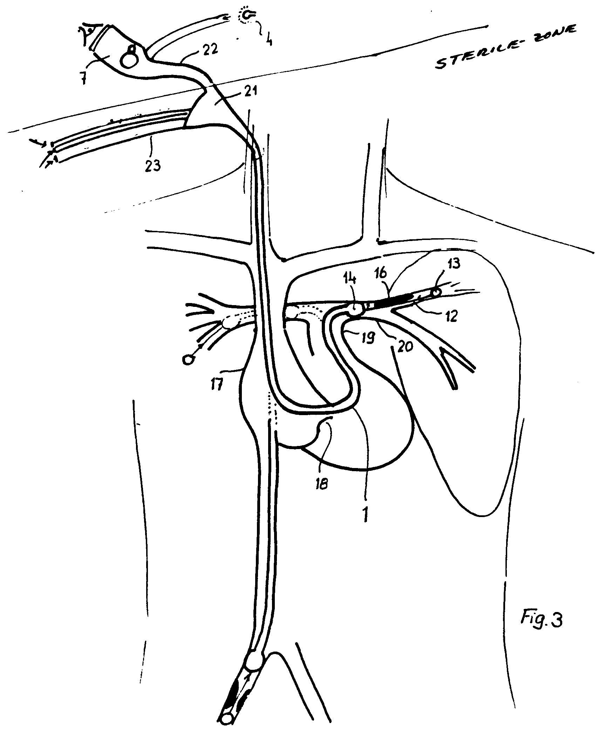

Parallel zu den Leitern 2, 3, 8 und dem Spülkanal 9 verläuft ein Saugkanal 10, dessen Durchmesser zwischen 6 mm und 8 mm liegt. Dieser Saugkanal mündet ebenfalls an der Spitze 5. Parallel zu den vorgenannten Kanälen verläuft noch ein Arbeitskanal 11, dessen Durchmesser etwa 1,5 mm beträgt. Durch den ebenfalls an der Spitze 5 mündenden Arbeitskanal 11 verläuft ein hohler Katheder 12, der über die Spitze 5 übersteht und an seinem über die Spitze 5 überstehenden Ende einen dilatierbaren Ballon 13 aufweist, der in Fig. 1 in kollabiertem Zustand gezeigt ist.

Das patientenseitige Endteil 6 ist von einem dilatierbaren Ballon 14 umgeben, der in Fig. 1 im flüssigkeitsgefüllten Zustand gezeigt ist. Im Inneren dieses Ballons 14 mündet ein weiterer Kanal 15, über den der Ballon 14 gefüllt oder entleert werden kann. Dieser weitere Kanal weist einen Durchmesser von etwa 1 mm auf.

Wie die Fig. 2 zeigt, ist der Saugkanal 10 exzentrisch zum Außendurchmesser des Schlauches 1 angeordnet. Die Leiter 2, 3, 8 und die Kanäle 9, 11, 15 sind in dem dickeren Bereich der Wand des Schlauches 1 angeordnet.

Zur Beseitigung eines Embolus 16 in einem Ast der linken Pulmonalarterie wird an der rechten Halsvene ein Schnitt angebracht und dort der Endoskopschlauch gegebenenfalls über eine Schleuse eingeführt. Der Ballon 14 und ebenso der Ballon 13 des bereits vorgeschobenen Fogarthy-Katheders 12 befinden sich in kollabiertem Zustand. Der Schlauch wird in den rechten Herzvorhof eingebracht, durch die Herzklappe 18 gelenkt und über die rechte Herzkammer in die Pulmonalarterie 19 vorgeschoben. Dies erfolgt unter Röntgenkontrolle. Wird nunmehr über den Spülkanal 9 ein Kontrastmittel in die Pulmonalarterie eingebracht, dann ist mittels der Röntgenkontrolle der Sitz des Embolums 16 erkennbar. Sitzt dieses Embolum 16 in einem Ast der linken Pulmonalarterie, dann wird das Endteil 6 des Endoskopschlauches 1 in die linke Pulmonalarterie gelenkt.

In der linken Pulmonalarterie 20 wird nunmehr der Ballon 14 dilatiert, indem über den Kanal 15 Flüssigkeit zugeführt wird. Hierdurch legt sich der Ballon 14 an die Innenwand der Arterie 20. Über den Spülkanal 9 wird Spülflüssigkeit zugeführt, welche die Spitze 5 von Blut freispült. Unter Lichtbeobachtung wird der Fogarthy-Katheder 12 mit seinem noch kollabierten Ballon 13 durch das Hindernis geschoben und ebenfalls dilatiert, indem über das Innere des Katheders 12 Flüssigkeit dem Ballon 13 zugeführt wird. Der Thrombus 16 kann nunmehr über den Saugkanal 10 abgesaugt werden. Handelt es sich um einen festen Thrombus, ist es möglich, über den Spülkanal 9 eine Lyseflüssigkeit als Weichmacher zuzuführen.

Bei festsitzenden Thromben ist es möglich, das Gefäß mittels des Ballons 13 des Fogarthy-Katheders 12 freizuräumen. In diesem Fall wird der Katheder 12 mit seinem dilatierten Ballon 13 in Richtung der Spitze 5 gezogen, nachdem zuvor der Katheder 12 mit kollabiertem Ballon 13 den Thrombus durchstoßen hat. Auf diese Weise können die Gefäße einzeln unter Licht mittels des Katheders 12 freigeräumt werden, was auch beispielsweise bei den Beinvenen möglich ist, wie dies die Fig. 3 andeutet.

Die vorgenannten Arbeiten werden unter Beobachtung des vom Bildleiter 8 gelieferten Bildes durchgeführt.

Außerhalb des Patienten weist der Endoskopschlauch 1 eine Gabelung 21 auf. In dem einen Zweig 22 der Gabelung verlaufen die Licht- und Bildleiter 2, 3, 8 sowie die nichtdargestellten Bowdenzüge zur Steuerung des patientenseitigen Endteils 6 des Endoskopschlauches 1. Im anderen, sterilen Zweig 23 der Gabelung 21 verlaufen die vorgenannten Kanäle 9, 10, 11, 15. Diese Kanäle enden an einem gemeinsamen Kupplungsstück 24. Am Kupplungsstück 24 münden die Kanäle 9, 10, 15 in einer Ebene, während der Kanal 11 an einem Ansatz 25 endet, durch den der Fogarthy-Katheder 12 geführt ist. Dieser Katheder 12 endet beispielsweise an einer Spritze, durch die über den Kanal 26 der Ballon 13 gefüllt und entleert werden kann. Auf das Kupplungsstück 24 ist ein weiteres Kupplungsstück aufschiebbar, das den Kanälen 9, 10, 15 entsprechende Kanäle aufweist. Der dem Kanal 10 entsprechende Kanal führt zu einem Sauger, über den dem Kanal entsprechenden Kanal wird Spül- oder Kontrastflüssigkeit bzw. ein Medikament zugeführt, während über den dem Kanal 15 entsprechenden Kanal die Flüssigkeit zu- oder abgeführt wird, mit der der Ballon 14 gefüllt bzw. entleert wird.

Der oder die Lichtleiter 2, 3 sowie der Bildleiter 8 können in einem gemeinsamen Kanal des Endoskopschlauchs 1 angeordnet sein. Es ist weiterhin möglich, den einen Zweig 22 zusammen mit den Licht- und Bildleitern 2, 3, 8 vom Endoskopschlauch 1 zu lösen, der nach Gebrauch weggeworfen wird. Die Licht- und Bildleiter 2, 3, 8 werden dann in einem neuen sterilen Schlauch 1 eingesetzt und der Zweig 22 auf diesem Schlauch aufgesteckt.

## Patentansprüche

1. Endoskop mit mindestens einem Lichtleiter (2, 3), einem Bildleiter (8), einem Spülkanal (9) und einem an eine Saugpumpe anschließbaren Saugkanal (10), wobei der Licht- und der Bildleiter (2, 3, 8) sowie der Spül- und der Saugkanal (9, 10) an der Spitze (5) des patientenseitigen Endteils (6) eines Endoskopschlauches (1) münden, das Endteil (6) von einem ersten dilatierbaren Ballon (14) umgeben ist und ein über die Spitze (5) überstehender Katheter (12) vorgesehen ist, der an seinem patientenseitigen Ende einen zweiten dilatierbaren Ballon (13) aufweist, **dadurch gekennzeichnet,** daß der Endoskopschlauch (1) an seinem dem patientenseitigen Endteil (6) gegenüberliegenden Ende eine Gabelung (21) aufweist und in dem einen Zweig (22) der Gabelung (21) die Licht- und Bildleiter (2, 3, 8) verlaufen, während der andere Zweig (23) der Gabelung (21) den Saugkanal (10), den Spülkanal (9) und mindestens einen Arbeitskanal (11) aufnimmt, in welchem der Katheter (12) verläuft, das patientenseitige Endteil (6) des Endoskopschlauches (1) über Bowdenzüge schwenkbar ist und der eine Zweig (22) zusammen mit den Licht- und Bildleitern (2, 3, 8) vom Endoskopschlauch (1) lösbar ist.

2. Endoskop nach Anspruch 1, **dadurch gekennzeichnet,** daß der Saugkanal (10) einen zum Spülkanal (9) größeren Durchmesser aufweist.

3. Endoskop nach einem der voranstehenden Ansprüche, **dadurch gekennzeichnet,** daß der Katheter (12) in dem Arbeitskanal (11) des Endoskopschlauches (1) verschiebbar geführt und zur Zufuhr der Dilatationsflüssigkeit hohl ausgebildet ist.

4. Endoskop nach einem der voranstehenden Ansprüche, **dadurch gekennzeichnet,** daß im Endoskopschlauch (1) ein weiterer Kanal (15) verläuft, der im Inneren des ersten Ballons (14) mündet und über den Dilatationsflüssigkeit dem ersten Ballon (14) zuführbar ist.

5. Endoskop nach einem der voranstehenden Ansprüche, **dadurch gekennzeichnet,** daß der andere Zweig (23) in einem für die Kanäle gemeinsamen Kupplungsstück (24) endet.

6. Endoskop nach einem der voranstehenden Ansprüche, **dadurch gekennzeichnet,** daß der Endoskopschlauch (1) einen kreisförmigen Querschnitt aufweist, der Saugkanal (10) exzentrisch darin angeordnet ist und die Licht- und Bildleiter (2, 3, 8) sowie die übrigen Kanäle (9, 11, 15) im Bereich des dickeren Teils der Schlauchwand verlaufen.

7. Endoskop nach einem der voranstehenden Ansprüche, **dadurch gekennzeichnet,** daß das Endteil (6) um etwa 120° schwenkbar und etwa 10 cm lang ist.

8. Endoskop nach einem der voranstehenden Ansprüche, **dadurch gekennzeich**net, daß die Ballons (13, 14) auf etwa 4 cm Durchmesser dilatierbar sind.

9. Endoskop nach einem der voranstehenden Ansprüche, **dadurch gekennzeichnet,** daß der Saugkanal (10) einen Durchmesser von etwa 8 mm und der Spülkanal (9) einen Durchmesser von etwa 1.0 mm aufweist.

10. Endoskop nach einem der voranstehenden Ansprüche, **dadurch gekennzeichnet,** daß Licht- und Bildleiter (2, 3, 8) in einem gemeinsamen Kanal des Endoskopschlauches (1) geführt sind.

## Claims

1. An endoscope having at least one light-conductor (2, 3), one image conductor (8), one lavage channel (9) and one suction channel (10) which may be connected to a suction pump, in which the light and image conductors (2, 3, 8) as well as the lavage and suction channels (9, 10) emerge at the tip (5) of the part (6) of an endoscope tube (1) at the end next the patient, the end part (6) is surrounded by a first dilatable balloon (14) and a catheter (12) is provided which extends beyond the tip (5) and exhibits at the end of it next the patient a second dilatable balloon (13), **characterized in that**
at the end of it remote from the end part (6) next the patient the endoscope tube (1) exhibits a bifurcation (21) and the light and image conductors (2, 3, 8) run in the one branch (22) of the bifurcation (21) whilst the other branch (23) of the bifurcation (21) receives the suction channel (10), the flushing channel (9) and at least one work channel (11) for the catheter (12) to run in, and the end part (6) of the endoscope tube (1) next the patient may be swivelled by a Bowden wire and the one branch (22) together with the light and image conductors (2, 3, 8) is detachable from the endoscope tube (1).

2. An endoscope as in Claim 1, characterized in that
the suction channel (10) exhibits a diameter larger than that of the lavage channel (9).

3. An endoscope as in one of the preceding Claims,
characterized in that the catheter (12) is guided to be able to shift in the work channel (11) of the endoscope tube (1) and is made hollow for feeding the liquid for the dilation.

4. An endoscope as in one of the preceding Claims,
characterized in that a further channel (15) runs in the endoscope tube (1) to emerge inside the first balloon (14), and liquid may be fed through it for dilating the first balloon (14).

5. An endoscope as in one of the preceding Claims,
characterized in that the other branch (23) ends in a coupling-piece (24) common to the channels.

6. An endoscope as in one of the preceding Claims,
characterized in that the endoscope tube (1) exhibits a circular cross-section, the suction channel (10) is arranged offcentre in it and the light and image conductors (2, 3, 8) as well as the remaining channels (9, 11, 15) run in the region of the thicker part of the wall of the tube.

7. An endoscope as in one of the preceding Claims,
characterized in that the end part (6) is able to swivel through about 120° and is about 10 cm long.

8. An endoscope as in one of the preceding Claims,
characterized in that the balloons (13, 14) may be dilated to a diameter of about 4 cm.

9. An endoscope as in one of the preceding Claims,
characterized in that the suction channel (10) exhibits a diameter of about 8 mm and the lavage channel (9) a diameter of about 1.0 mm.

10. An endoscope as in one of the preceding Claims,
characterized in that the light and image conductors (2, 3, 8) are led through a common channel in the endoscope tube (1).

## Revendications

1. Endoscope comportant au moins un conduit de lumière (2, 3) et un conduit d'images (8), un canal de lavage (9) et un canal d'aspiration (10) pouvant être raccordé à une pompe d'aspiration, sachant que le conduit de lumière et le conduit d'images (2, 3, 8) ainsi que le canal de lavage et le canal d'aspiration (9, 10) débouchent à la pointe (5) de la partie terminale (6) d'un tube d'endoscope (1) située du côté du patient, que la partie terminale (6) est entourée par un premier ballon (14) dilatable et qu'un cathéter (12) faisant saillie au-dessus de la pointe (5), qui présente à son extrémité située du côté du patient un deuxième ballon (13) dilatable, est prévu, caractérisé en ce que le tube de l'endoscope (1) présente, à son extrémité opposée à la partie terminale (6) située du côté du patient, une bifurcation (21), en ce que les conduits de lumière et d'images (2, 3, 8) s'étendent dans l'une des branches (22) de la bifurcation (21), tandis que l'autre branche (23) de la bifurcation (21) contient le canal d'aspiration (10), le canal de lavage (9) et au moins un canal de travail (11) dans lequel s'étend le cathéter (12), en ce que la partie d'extrémité (6) du tube de l'endoscope (1) située du côté du patient peut pivoter par l'intermédiaire de câbles Bowden, et en ce que l'une des branches (22) peut être détachée du tube de l'endoscope (1) avec les conduits de lumière et d'images (2, 3, 8).

2. Endoscope selon la revendication 1, caractérisé en ce que le canal d'aspiration (10) présente un diamètre supérieur à celui du canal de lavage (9).

3. Endoscope selon l'une des revendications précédentes, caractérisé en ce que le cathéter (12) est guidé de manière mobile dans le canal de travail (11) du tube de l'endoscope (1) et est réalisé creux pour amener le liquide dilatateur.

4. Endoscope selon l'une des revendications précédentes, caractérisé en ce que dans le tube de l'endoscope (1) s'étend un autre canal (15) qui débouche à l'intérieur du premier ballon (14) et par le biais duquel du liquide dilatateur peut être amené au premier ballon (14).

5. Endoscope selon l'une des revendications précédentes, caractérisé en ce que l'autre branche (23) se termine dans une pièce d'accouplement (24) commune aux canaux.

6. Endoscope selon l'une des revendications précédentes, caractérisé en ce que le tube de l'endoscope (1) présente une section transversale circulaire, en ce que le canal d'aspiration (10) y est disposé de façon excentrée, et en ce que les conduits de lumière et d'images (2, 3, 8) ainsi que les autres canaux (9, 11, 15) s'étendent dans la zone de la partie plus épaisse de la paroi du tube.

7. Endoscope selon l'une des revendications précédentes, caractérisé en ce que la partie terminale (6) peut pivoter d'environ 120° et a approximativement 10 cm de long.

8. Endoscope selon l'une des revendications précédentes, caractérisé en ce que les ballons (13, 14) sont dilatables jusqu'à atteindre environ 4 cm de diamètre.

9. Endoscope selon l'une des revendications précédentes, caractérisé en ce que le canal d'aspiration (10) présente un diamètre d'environ 8 mm et le canal de lavage (10) un diamètre d'environ 1,0 mm.

10. Endoscope selon l'une des revendications précédentes, caractérisé en ce que les conduits de lumière et d'images (2, 3, 8) sont guidés dans un canal commun du tube de l'endoscope (1).
